# EUROPÄISCHE PATENTSCHRIFT

(11) **EP 1 607 106 B1**
(45) Veröffentlichungstag und Bekanntmachung des Hinweises auf die Patenterteilung: **09.02.2011**
(21) Anmeldenummer: 05011067.5
(22) Anmeldetag: 23.05.2005
(51) Int. Cl.: A61L 2/20, B65B 55/10, B67C 7/00

(54) **Gefässbehandlungsmaschine zur Sterilisation von Behältern mittels H202**
Apparatus for sterilzing containers using H2O2
Dispositif pour la stérilisation de récipients en utilisant H2O2

(30) Priorität: 19.06.2004 DE 102004029803
(43) Veröffentlichungstag der Anmeldung: 21.12.2005
(73) Patentinhaber: KHS GmbH, Juchostrasse 20 44143 Dortmund (DE)
(72) Erfinder: Stienen, Thomas, 59425 Unna (DE)

(56) Entgegenhaltungen:
- WO-A-03/103726
- DE-A1- 2 339 005
- NL-A- 9 201 556
- US-A- 4 214 867
- US-A- 4 742 667
- US-A- 6 120 730
- US-A1- 2003 194 347
- PATENT ABSTRACTS OF JAPAN Bd. 2000, Nr. 09, 13. Oktober 2000 (2000-10-13) & JP 2000 153285 A (SHINSANSO KAGAKU KK), 6. Juni 2000 (2000-06-06)
- HASAN M A ET AL: "Promotion of the hydrogen peroxide decomposition activity of manganese oxide catalysts" APPLIED CATALYSIS A: GENERAL, ELSEVIER SCIENCE, AMSTERDAM, NL, Bd. 181, Nr. 1, 3. Mai 1999 (1999-05-03), Seiten 171-179, XP004271777 ISSN: 0926-860X

## Beschreibung

Die vorliegende Erfindung betrifft eine Gefäßbehandlungsmaschine gemäß dem Oberbegriff des Anspruches 1. Vorteilhafte Weiterbildungen sind in den Unteransprüchen angegeben.

Beispielsweise in der Getränkeindustrie, d. h. beim Abfüllen von Getränken in Flaschen oder dgl. Behälter ist es vielfach erforderlich, diese Behälter vor dem Füllen zur Erzielung der erforderlichen Keimfreiheit und damit Haltbarkeit des abgefüllten Produktes zumindest an deren Innenflächen zu sterilisieren. Bekannt ist hierbei z.B. die sogenannte H₂O₂-Sterilisation. Bei einem dieser Verfahren wird z.B. flüssiges H₂O₂ zunächst feinverstäubt einem Luftstrom beigemischt, wobei es sich in der Regel um einen Strom steriler Luft handelt. Anschließend wird dieses H₂O₂-Luft-Gemisch einem Verdampfer zugeführt, in welchem das noch flüssige H₂O₂ vollständig verdampft wird. Nachfolgend wird dieses Dampf-Luft-Gemisch in die zu sterilisierenden Behälter eingeleitet, wo das H₂O₂ sofort an den kalten Behälterinnenwandungen kondensiert und dort einen gleichmäßigen Flüssigkeitsfilm bildet. Zur nun folgenden Aktivierung des H₂O₂, d.h. zur Auslösung der Zersetzung des H₂O₂, ist es erforderlich, dieses auf eine bestimmte Temperatur zu erwärmen, bzw. diesem eine bestimmte Wärmemenge zuzuführen, welche in der Regel durch ein Aktivierungsmedium auf das H₂O₂ übertragen wird. Bei diesem Aktivierungsmedium handelt es sich in den meisten Fällen um in die Behälter eingeblasene sterile Warmluft, welche bis auf die zur Aktivierung des H₂O₂ erforderliche Temperatur erwärmt wurde.

US-4,742,667 und US-6,120,730 beschreiben Verfahren und Geräte zur Sterilisation von Gefäßen mittels H₂O₂.

Im Laufe des Zersetzungsprozesses zerfällt das H₂O₂ in Wasser und freie Radikale, nämlich atomaren Sauerstoff 0 und HO-Gruppen, welche die eigentliche Sterilisation im Wesentlichen durchführen. Nach dem Abschluss der Sterilisation werden die Behälter mittels Spül- und/oder Trockenluft ausgeblasen und getrocknet.

JP-2000153285 beschreibt das Aktivieren von H₂O₂ mittels Katalysatoren von den Sterilisationseffekt zu erhöhen.

Berichtet wurde ebenfalls von Verfahren, bei denen bereits in Dampfform vorliegendes H₂O₂ durch weitere Temperaturerhöhungen aktiviert bzw. in die Zersetzung überführt wurde.

Die bekannten, mit Temperaturaktivierung arbeitenden Verfahren weisen zahlreiche Nachteile auf.

In erster Linie ist der hohe Zeitbedarf für die Aktivierung bzw. Zersetzung des H₂O₂ in seine wirksamen Bestandteile zu nennen. Bei in der Praxis ausgeführten Anlagen wurden Zykluszeiten für Aktivierung, Sterilisation und anschließendes Spülen mit Spül- und/oder Trockenluft von bis zu 20 Sekunden ermittelt, was bei den kundenseitig gewünschten hohen Anlagenleistungen für Gefäßfüllmaschinen u.a. zur Folge hat, dass einer Gefäßfüllmaschine zwei oder sogar drei Gefäßbehandlungsmaschinen bzw. Sterilisationsvorrichtungen zugeordnet werden müssen, was einen überaus großen Kostenaufwand bedeutet.

Ebenfalls ist es bei der Temperaturaktivierung des H₂O₂ von großem Nachteil, dass die immer häufiger eingesetzten Kunststoffflaschen einer Temperaturbeschränkung unterliegen, wodurch die maximale Aktivierungstemperatur begrenzt wird. Aufgrund der sich mit steigender Aktivierungstemperatur überproportional verkürzenden Zersetzungszeit des H₂O₂ ist eine möglichst hohe Aktivierungstemperatur gewünscht, übersteigt diese jedoch die durch die Kunststoffflaschen vorgegebene maximale Temperatur, so kommt es z.B. durch unerwünschte Verkleinerungen und/oder Verformungen der Kunststoffflaschen zu kostenträchtigen Schädigungen des Flaschenmaterials.

Ein weiterer Nachteil der Temperaturaktivierung besteht darin, dass es, insbesondere bei ausschließlicher Sterilisation der Innenwandungen der Kunststoffflaschen zu mehr oder weniger großen Temperaturunterschieden in den Wandungen der Kunststoffflaschen kommen kann, wodurch es z.B. durch die Bildung von Rissen zu einer verminderten Haltbarkeit und zu Beeinträchtigungen hinsichtlich der optischen Anmutung der Behälter kommen kann.

Aufgabe und Ziel der vorliegenden Erfindung ist es, die oben dargestellten Nachteile bei gleichzeitiger Erzielung einer deutlich verkürzten Aktivierungs- bzw. Zersetzungszeit für das H₂O₂ sicher zu vermeiden. Dazu sieht die Erfindung vor, die Zersetzung des H₂O₂ durch den Einsatz mindestens eines Katalysators zu beschleunigen, wobei die erforderliche Zersetzungstemperatur ebenfalls reduziert wird.

Im Nachfolgenden wird die vorliegende Erfindung anhand eines Ausführungsbeispieles näher erläutert.

Die Erfindung ist in den Ansprüchen definiert.

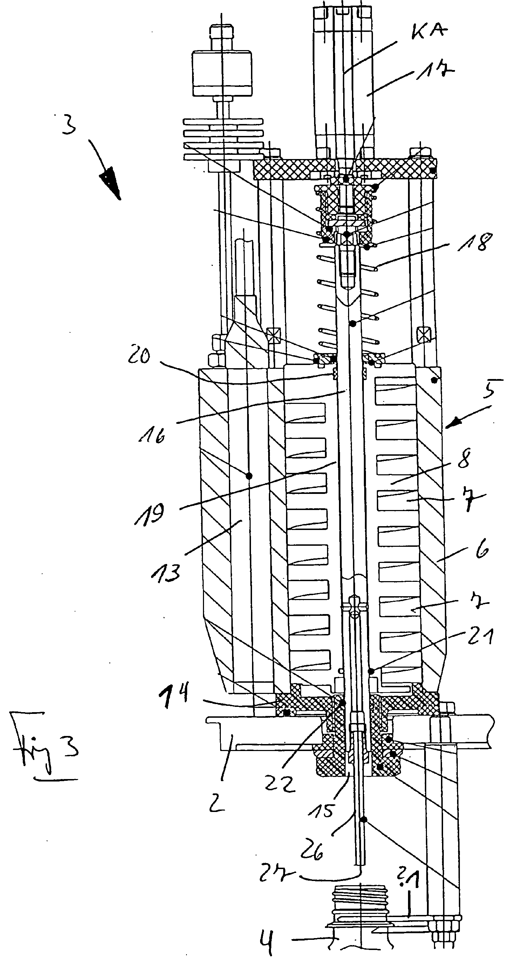

Im Einzelnen zeigt die
- Figuren 1 und 3: in vereinfachten Schnittdarstellungen Sterilisatorköpfe, und die
- Figur 2: in einer vereinfachten Draufsicht eine als Rundläufer ausgebildete Gefäßbehandlungsmaschine.

Weiterbildungen, Vorteile und Anwendungsmöglichkeiten der Erfindung ergeben sich aus der nachfolgenden Beschreibung von Ausführungsbeispielen und der Zeichnung. Dabei bilden alle beschriebenen und/oder bildlich dargestellten Merkmale für sich oder in beliebiger Kombination den Gegenstand der Erfindung, unabhängig von ihrer Zusammenfassung in den Ansprüchen oder deren Rückbeziehung. Gleichzeitig wird der Inhalt der Ansprüche zu einem Bestandteil der Beschreibung gemacht.

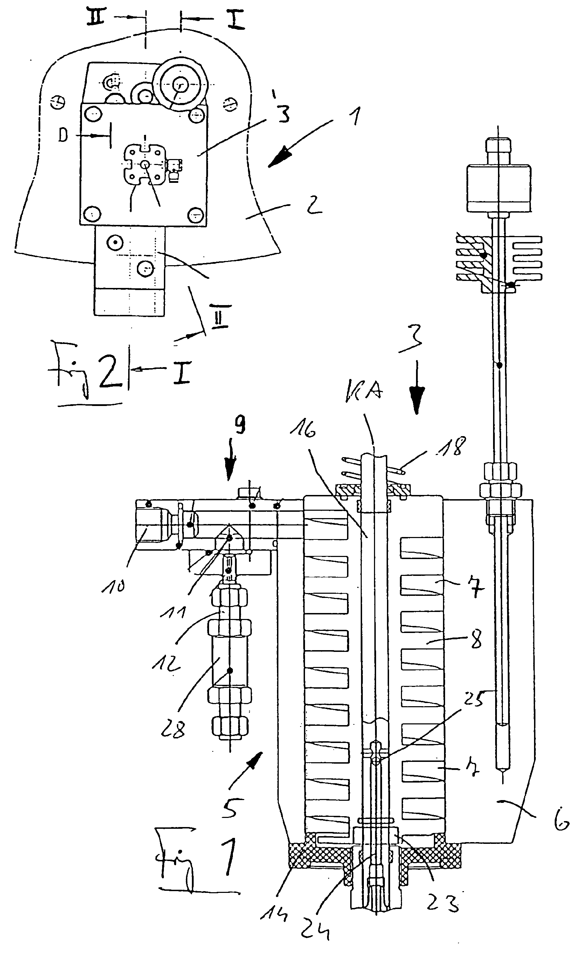

Die Figur 1 zeigt in einer vereinfachten Darstellung einen Sterilisatorkopf 3, wie er zu mehreren sowohl an Rundläufer-, als auch an Linearmaschinen zur Desinfektion von Behältern angeordnet sein kann.

Der als Ausführungsbeispiel zu verstehende Sterilisatorkopf 3 dient zur H₂O₂ - Sterilisation von Behältern, z.B. temperaturempfindlichen PET-Flaschen.

Unter den Sterilisatorköpfen 3, die z.B. in gleichmäßigen Winkelabständen am Umfang des Rotors 2 angeordnet sind, ist jeweils ein Behälter- oder Flaschenträger vorgesehen, an dem die jeweilige, zu sterilisierende Flasche z.B. hängend gehalten ist, und zwar achsgleich mit einer vertikalen Sterilisatorkopfachse KA, sodass beim Aktivieren des Sterilisatorkopfes 3 ein Strahl des heißen Sterilisationsmedium in den Innenraum der sich unterhalb des Sterilisationskopfes 3 befindlichen zu sterilisierenden Flasche 4 Flasche ausgebracht werden kann.

Der Sterilisatorkopf 3 besteht aus einem Erhitzer oder Wärmetauscher 5 mit einem Gehäuse 6, in welchem u. a. ein die Achse KA schraubenartig umschließender Strömungs- oder Heizkanal 7 ausgebildet ist, und zwar bei der dargestellten Ausführungsform dadurch, dass in eine kreiszylinderförmige, achsgleich mit der Achse KA ausgebildete durchgehende Öffnung des Gehäuses 6 ein Kern 8 eingesetzt ist, der an seinem Umfang eine den Strömungskanal 7 bildende schraubenförmige Nut aufweist.

Das in der Figur 1 obere Ende des nach außen hin geschlossenen Strömungskanal 7 steht mit einer Sprüheinrichtung 9 in Verbindung, der über den Anschluss 10 sterile Druckluft zugeführt wird und die eine Sprühdüse 11 besitzt, der über den Anschluss 12 und eine mit diesem Anschluss verbundene, nicht dargestellte Pumpe, beispielsweise einer Membran-Pumpe, H₂O₂ zugeführt wird, welches dann zur Bildung des H₂O₂-Luft-Aerosols über die Sprühdüse 11 als fein versprühter Strahl oder Nebel in den die Sprüheinrichtung 9 durchströmenden Luftstrom eingebracht wird, sodass dieses aus Luft und H₂O₂ bestehende Aerosol dann in den Strömungskanal 7 des Wärmetauschers 5 gelangt.

Durch eine nicht dargestellte Heizeinrichtung, welche z.B. von einer elektrischen Heizpatrone mit Thermofühler gebildet sein kann, werden der Wärmetauscher 5 und insbesondere auch der Kern 8 beheizt.

Das untere Ende des Gehäuses 6 wird durch geeignete Elemente gebildet, welche es gestatten, das den H₂O₂ -Dampf enthaltende Dampf-Luft-Gemisch kontinuierlich oder aber auch diskontinuierlich direkt oder indirekt in die zu sterilisierenden Flaschen 4 abzugeben. Dazu kann beispielsweise ein Rohr 26 dienen, welches mit seiner Abgabeöffnung 27 zumindest zeitweise in die Flaschen 4 hineinreichen kann, oder aber auch mit seiner Abgabeöffnung 27 unmittelbar vor der Mündung der Flaschen 4 positioniert sein kann.

Der Wärmetauscher wird durch die Heizeinrichtung erwärmt, und zwar auf eine Temperatur, welche unter Berücksichtigung aller relevanter Parameter, wie z.B. Volumen-und/oder Massenstrom des H₂O₂ enthaltenden Aerosols, Ausgangstemperatur des Aerosols, spezifischer Wärmekapazität usw. sicherstellt, dass das im Aerosol enthaltene H₂O₂ spätestens beim Verlassen des Wärmetauschers 5 im gewünschten Maße verdampft ist.

Bei bekannten, dem Stand der Technik entsprechenden Ausgestaltungen ist es üblich, den Wärmetauscher 5 z.B. auf Temperaturen in der Größenordnung von 130 - 150°C zu erwärmt, wodurch häufig zusätzliche Maßnahmen zur Vermeidung einer Überhitzung der Flaschen 4 erforderlich werden.
Erfindungsgemäß ist vorgesehen, innerhalb des Weges des H₂O₂ bzw. des Weges des, das H₂O₂ enthaltenden Aerosols mindestens einen Katalysator zur Auslösung und Unterstützung des Zersetzungsprozesses des H₂O₂ bei niedrigen Temperaturen anzuordnen.

Durch die erfindungsgemäße Anordnung des mindestens einen Katalysators wird erreicht, dass die zur Auslösung des Zersetzungsprozesses des H₂O₂ erforderliche Aktivierungsenergie herabgesetzt wird, so dass die Zersetzung schon bei z.B. Raumtemperatur mit großer Geschwindigkeit abläuft, wodurch die wirksamen freien Radikale nicht erst durch zeitaufwändige thermische Aktivierung innerhalb der Flaschen 4 erzeugt werden müssen sondern direkt in diese eingebracht werden können.

Als chemisch wirksames Material für den mindestens einen Katalysator könne alle in der Technik bekannten, für diesen Anwendungsfall geeigneten Materialien verwendet werden. So z.B. die bekanntermaßen besonders gut geeigneten Edelmetalle der Platingruppe, aber auch z.B. Braunstein oder weitere Stoffe.

Dabei bietet es sich an, diese Materialien in einer Form zu verwenden, welche eine besonders große wirksame Oberfläche aufweist. Insbesondere ist die Verwendung von aus Drähten kleiner Durchmesser hergestellter Drahtgeflechte und/oder durch pulvermetallurgische Verfahren hergestellter Katalysatorelemente zu bevorzugen.

Beispielhaft, aber den Umfang der vorliegenden Erfindung nicht beschränkend seien nachfolgend einigen Anordnungspositionen für den mindestens einen Katalysator genannt.

Es ist vorgesehen, das Innere des Rohres 26 zumindest teilweise mit einem Katalysator auszufüllen.

Außerdem ist vorgesehen, einen vorzugsweise aus porösem Material bestehenden Katalysator als Abgabeelement für das sich zersetzende bzw. bereits zersetzte H₂O₂ auszubilden, wobei die Mantelfläche dieses Abgabeelementes luftdicht ausgebildet ist. Die Mündung dieses Abgabe-Katalysatorelementes endet vor der Mündung der Behälter 4 oder aber auch innerhalb des Behälters 4.

Ebenfalls ist vorgesehen, die Oberfläche des Wärmetauschers 5 zumindest teilweise mit einem als Katalysator wirksamen Material zu beschichten, bzw. den Wärmetauscher 5 zumindest teilweise aus einem solchen Material herzustellen.

Des Weiteren ist vorgesehen, jedem Sterilisatorkopf bzw. jeder Behandlungsstation mindestens einen Katalysator direkt zuzuordnen, und/oder mindestens einen Katalysator einer Gruppe von Sterilisatorköpfen zuzuordnen, und/oder allen Sterilisatorköpfen einer Sterilisationsmaschine gemeinsam mindestens einen Katalysator zuzuordnen.

In einer weiteren Ausgestaltung der vorliegenden Erfindung ist vorgesehen, den mindestens einen Katalysator als (zusätzliches) Heizelement auszubilden, wodurch sich weitere Möglichkeiten zur gezielten Steuerung des Zersetzungsprozesses des H₂O₂ ergeben.

## Patentansprüche

1. Gefäßbehandlungsmaschine, insbesondere zur Sterilisation von aus Kunststoff, Metall oder Glas bestehenden Flaschen, Dosen oder dergleichen Behälter mittels H₂O₂, ausgeführt in Rundläufer- oder Linearbauweise, **dadurch gekennzeichnet, dass** im Strömungsweg des flüssigen und/oder gasförmigen H₂O₂ von seiner Quelle zu dem zu behandelnden Behälter (4) mindestens ein, die Zersetzung des H₂O₂ fördernder Katalysator angeordnet ist.

2. Gefäßbehandlungsmaschine nach Anspruch 1, **dadurch gekennzeichnet, dass** der mindestens eine Katalysator innerhalb eines, das H₂O₂ und/oder dessen Zersetzungsprodukte in oder an die Behälter (4) leitenden Rohres (26) angeordnet ist.

3. Gefäßbehandlungsmaschine nach einem der vorhergehenden Ansprüche, **dadurch gekennzeichnet, dass** der Katalysator als ein, das H₂O₂ und/oder dessen Zersetzungsprodukte in oder an die Behälter (4) leitendes, aus porösem Material bestehendes Rohr (26) mit ausgebildet ist.

4. Gefäßbehandlungsmaschine nach einem der vorhergehenden Ansprüche, **dadurch gekennzeichnet, dass** die Oberfläche eines, zur Erwärmung dienenden Wärmetauschers (5) zumindest teilweise als Katalysator ausgebildet ist.

5. Gefäßbehandlungsmaschine nach Anspruch 4, **dadurch gekennzeichnet, dass** der Wärmetauscher (5) zumindest teilweise aus katalytisch wirksamen Material besteht.

6. Gefäßbehandlungsmaschine nach einem der vorhergehenden Ansprüche, **dadurch gekennzeichnet, dass** der mindestens eine Katalysator aus Edelmetallen der Platingruppe und/oder Braunstein besteht.

7. Gefäßbehandlungsmaschine nach einem der vorhergehenden Ansprüche, **dadurch gekennzeichnet, dass** der mindestens eine Katalysator mittels pulvermetallurgischer Verfahren hergestellt ist.

8. Gefäßbehandlungsmaschine nach einem der vorhergehenden Ansprüche, **dadurch gekennzeichnet, dass** der mindestens eine Katalysator als Drahtgeflecht ausgebildet ist, wobei das Drahtgeflecht aus einem Draht mit kleinem Durchmesser gebildet ist.

9. Gefäßbehandlungsmaschine nach einem der vorhergehenden Ansprüche, **dadurch gekennzeichnet, dass** der mindestens eine Katalysator als Heizelement für das H₂O₂ bzw. dessen Zersetzungsprodukte ausgebildet ist.

10. Gefäßbehandlungsmaschine nach einem der vorhergehenden Ansprüche, **dadurch gekennzeichnet, dass** jedem an der Gefäßbehandlungsmaschine vorgesehenem Sterilisatorkopf(3) mindestens ein Katalysator unmittelbar zugeordnet ist.

11. Gefäßbehandlungsmaschine nach einem der vorhergehenden Ansprüche, **dadurch gekennzeichnet, dass** einer Gruppe der an der Gefäßbehandlungsmaschine vorgesehenen Sterilisatorköpfe(3) mindestens ein Katalysator unmittelbar zugeordnet ist.

12. Gefäßbehandlungsmaschine nach einem der vorhergehenden Ansprüche, **dadurch gekennzeichnet, dass** allen an der Gefäßbehandlungsmaschine vorgesehenen Sterilisatorköpfen (3) gemeinsam mindestens ein Katalysator unmittelbar zugeordnet ist.

13. Verfahren zum Betrieb einer Gefäßbehandlungsmaschine nach einem der vorhergehenden Ansprüche, **dadurch gekennzeichnet, dass** die Zersetzung des H₂O₂ bei einer Temperatur unterhalb der maximal zulässigen Temperatur der zu behandelnden Behälter erfolgt.

14. Verfahren zum Betrieb einer Gefäßbehandlungsmaschine nach einem der vorhergehenden Ansprüche, **dadurch gekennzeichnet, dass** die Zersetzung des H₂O₂ bei einer Temperatur unterhalb der Rückverformungs- und/oder Schrumpfungstemperatur von Kunststoffbehältern erfolgt.

15. Verfahren zum Betrieb einer Gefäßbehandlungsmaschine nach einem der vorhergehenden Ansprüche, **dadurch gekennzeichnet, dass** die Zersetzung des H₂O₂ bei Raumtemperatur erfolgt, vorzugsweise im Temperaturbereich von 15 bis 25 °C.

## Claims

1. Container treatment machine, especially for the sterilisation bottles, cans or similar containers consisting of plastic, metal or glass with H₂O₂ designed as carousel-type or linear machine, **characterised by** at least one catalytic converter, which promotes the decomposition of the H₂O₂, being arranged in the flow path of the liquid and/or gaseous H₂O₂ from its source to the container to be treated (4).

2. Container treatment machine according to Claim 1, **characterised by** at least one catalytic converter being arranged within one of the pipelines (26) conveying the H₂O₂ and/or its decomposition products into or to the containers (4).

3. Container treatment machine according to one of the preceding claims, **characterised by** the catalytic converter being designed as a pipe of porous material (26) conveying the H₂O₂ and/or its decomposition products into or to containers (4).

4. Container treatment machine according to one of the preceding claims, **characterised by** the surface of a heat exchanger (5), which serves the purpose of heating, is at least partially designed as a catalytic converter.

5. Container treatment machine according to Claim 4, **characterised by** heat exchanger (5) consisting at least partially of catalytic effective material.

6. Container treatment machine according to one of the preceding claims, **characterised by** at least one catalytic converter consisting of precious metals of the Platinum group and/or pyrolusite.

7. Container treatment machine according to one of the preceding claims, **characterised by** at least one catalytic converter being manufactured by means of a powder-metallurgical procedure.

8. Container treatment machine according to one of the preceding claims, **characterised by** at least one catalytic converter being designed as a wire mesh manufactured from small diameter wire.

9. Container treatment machine according to one of the preceding claims, **characterised by** at least one catalytic converter being designed as heating element for the H₂O₂ or for its decomposition elements.

10. Container treatment machine according to one of the preceding claims, **characterised by** at least one catalytic converter being directly assigned to each of the sterilisation heads (3) on the container treatment machine.

11. Container treatment machine according to one of the preceding claims, **characterised by** at least one catalytic converter being directly assigned to a group of sterilisation heads (3) on the container treatment machine.

12. Container treatment machine according to one of the preceding claims, **characterised by** at least one catalytic converter being directly assigned to all of the sterilisation heads (3) on the container treatment machine collectively.

13. Procedure for the operation of a container treatment machine according to one of the preceding claims, **characterised by** the decomposition of the H₂O₂ occurring at a temperature below the maximum allowable temperature of the containers which are being treated.

14. Procedure for the operation of a container treatment machine according to one of the preceding claims, **characterised by** the decomposition of the H₂O₂ occurring at a temperature below the recovery and/or contraction temperature of plastic containers.

15. Procedure for the operation of a container treatment machine according to one of the preceding claims, **characterised by** the decomposition of the H₂O₂ occurring at room temperature, preferably within the temperature range of 15 to 25 °C.

## Revendications

1. Machine de traitement de récipients, en particulier pour la stérilisation par H₂O₂ de bouteilles, canettes ou récipients similaires en matière plastique, métal ou verre, exécutée avec une alimentation rotative ou linéaire, **caractérisée en ce qu'**au moins un catalyseur activant la dégradation du H₂O₂ est prévu sur le trajet du courant de H₂O₂ liquide et/ou gazeux, entre sa source et le récipient (4) à traiter.

2. Machine de traitement de récipients selon la revendication 1, **caractérisée en ce que** le ou les catalyseurs sont prévus à l'intérieur d'une conduite (26) refoulant le H₂O₂ et/ou ses produits de dégradation dans ou contre les récipients (4).

3. Machine de traitement de récipients selon l'une des revendications précédentes, **caractérisée en ce que** le catalyseur est réalisé comme une conduite (26) en matériau poreux refoulant le H₂O₂ et/ou ses produits de dégradation dans ou contre les récipients (4).

4. Machine de traitement de récipients selon l'une des revendications précédentes, **caractérisée en ce que** la surface d'un échangeur de chaleur (5) à fonction d'échauffement est au moins partiellement réalisée comme catalyseur.

5. Machine de traitement de récipients selon la revendication 4, **caractérisée en ce que** l'échangeur de chaleur (5) est au moins partiellement en matériau catalytiquement actif.

6. Machine de traitement de récipients selon l'une des revendications précédentes, **caractérisée en ce que** le ou les catalyseurs sont en métaux nobles du groupe du platine et/ou en pyrolusite.

7. Machine de traitement de récipients selon l'une des revendications précédentes, **caractérisée en ce que** le ou les catalyseurs sont fabriqués par procédé de métallurgie des poudres.

8. Machine de traitement de récipients selon l'une des revendications précédentes, **caractérisée en ce que** le ou les catalyseurs sont réalisés comme treillis métallique, le treillis métallique étant formé d'un seul fil de diamètre réduit.

9. Machine de traitement de récipients selon l'une des revendications précédentes, **caractérisée en ce que** le ou les catalyseurs sont réalisés comme élément de chauffe pour le H₂O₂ ou ses produits de dégradation.

10. Machine de traitement de récipients selon l'une des revendications précédentes, **caractérisée en ce qu'**au moins un catalyseur est directement affecté à chaque tête de stérilisateur (3) prévue sur la machine de traitement de récipients.

11. Machine de traitement de récipients selon l'une des revendications précédentes, **caractérisée en ce qu'**au moins un catalyseur est directement affecté à un groupe des têtes de stérilisateur (3) prévus sur la machine de traitement de récipients.

12. Machine de traitement de récipients selon l'une des revendications précédentes, **caractérisée en ce qu'**au moins un catalyseur est directement affecté à toutes les têtes de stérilisateur (3) prévues sur la machine de traitement de récipients.

13. Procédé de fonctionnement d'une machine de traitement de récipients selon l'une des revendications précédentes, **caractérisé en ce que** la dégradation du H₂O₂ se produit à une température inférieure à la température maximale admissible des récipients à traiter.

14. Procédé de fonctionnement d'une machine de traitement de récipients selon l'une des revendications précédentes, **caractérisé en ce que** la dégradation du H₂O₂ se produit à une température inférieure à la température de compression et/ou de contraction de récipients en matière plastique.

15. Procédé de fonctionnement d'une machine de traitement de récipients selon l'une des revendications précédentes, **caractérisé en ce que** la dégradation du H₂O₂ se produit à température ambiante, de préférence dans une plage de température entre 15 et 25 °C.
